(19) Europäisches Patentamt
European Patent Office
Office européen des brevets

(11) **EP 3 084 345 B1**

## (12) FASCICULE DE BREVET EUROPEEN

(45) Date de publication et mention
de la délivrance du brevet:
**25.03.2020 Bulletin 2020/13**

(21) Numéro de dépôt: **14827756.9**

(22) Date de dépôt: **19.12.2014**

(51) Int Cl.:
***G01B 9/02*** *(2006.01)*    ***G02B 21/00*** *(2006.01)*

(86) Numéro de dépôt international:
**PCT/EP2014/078867**

(87) Numéro de publication internationale:
**WO 2015/092019 (25.06.2015 Gazette 2015/25)**

(54) **APPAREIL ET PROCEDE DE TOMOGRAPHIE OPTIQUE**

OPTISCHE TOMOGRAPHIEVORRICHTUNG UND VERFAHREN

DEVICE AND PROCESS OF OPTICAL TOMOGRAPHY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.12.2013 FR 1363234**

(43) Date de publication de la demande:
**26.10.2016 Bulletin 2016/43**

(73) Titulaires:
• **Centre National de la Recherche Scientifique**
**75016 Paris (FR)**
• **Institut d'Optique Graduate School**
**91127 Palaiseau cedex (FR)**
• **Universite Paris-Sud 11**
**91405 Orsay Cedex (FR)**

(72) Inventeur: **DUBOIS, Arnaud**
**F-91400 Orsay (FR)**

(74) Mandataire: **Osha Liang**
**2, rue de la Paix**
**75002 Paris (FR)**

(56) Documents cités:
**EP-A1- 2 447 754    FR-A1- 2 962 531**
**US-A1- 2002 021 491**

• **SEOKHAN KIM ET AL: "Simultaneous measurement of refractive index and thickness by combining low-coherence interferometry and confocal optics", OPTICS EXPRESS, vol. 16, no. 8, 14 avril 2008 (2008-04-14) , page 5516, XP055137008, ISSN: 1094-4087, DOI: 10.1364/OE.16.005516**
• **CHEN Y ET AL: "HIGH-RESOLUTION LINE-SCANNING OPTICAL COHERENCE MICROSCOPY", OPTICS LETTERS, OPTICAL SOCIETY OF AMERICA, US, vol. 32, no. 14, 15 juillet 2007 (2007-07-15), pages 1971-1973, XP001506730, ISSN: 0146-9592, DOI: 10.1364/OL.32.001971**
• **VAN VELTHOVEN ET AL: "Recent developments in optical coherence tomography for imaging the retina", PROGRESS IN RETINAL AND EYE RESEARCH, OXFORD, GB, vol. 26, no. 1, 14 décembre 2006 (2006-12-14), pages 57-77, XP005803711, ISSN: 1350-9462**
• **DUBOIS A ET AL: "Thermal-light full-field optical coherence tomography in the 1.2mum wavelength region", OPTICS COMMUNICATIONS, NORTH-HOLLAND PUBLISHING CO. AMSTERDAM, NL, vol. 266, no. 2, 15 octobre 2006 (2006-10-15), pages 738-743, XP028080898, ISSN: 0030-4018, DOI: 10.1016/J.OPTCOM.2006.05.016 [extrait le 2006-10-15]**

## Description

L'invention porte sur un appareil et un procédé de tomographie optique, destiné en particulier, mais pas exclusivement, à des applications biologiques et médicales, et notamment histologiques. D'autres applications possibles concernent, par exemple, la caractérisation de matériaux.

L'examen histologique de tissus prélevés par biopsie présente une grande utilité dans la pratique clinique, par exemple pour le diagnostic des tumeurs. Cependant, cette technique est lente et complexe à mettre en œuvre, car elle nécessite une biopsie, c'est-à-dire le prélèvement d'un échantillon du tissu à étudier, et sa découpe en fines tranches qui sont observées au microscope et analysées par un anatomopathologiste. La procédure complète nécessite en outre la fixation de l'échantillon, son occlusion dans une matrice et sa coloration. Cela pose problème surtout dans le cas d'examens pratiqués pendant des opérations chirurgicales, où la rapidité a une importance primordiale. En outre, l'étape de prélèvement peut être gênante, voire dangereuse pour le patient (cas du cerveau, par exemple). Pour cette raison, des techniques d'imagerie non intrusives - notamment optiques - ont été développées pour visualiser la structure interne des tissus biologiques - ou plus généralement d'objets semi-transparents. Pour pouvoir être compétitives avec les examens histologiques conventionnels, ces techniques doivent permettre d'accéder *in situ* à une profondeur de l'ordre du millimètre en-dessous de la surface du tissu et présenter une résolution de l'ordre du micromètre. La rapidité d'exécution, la simplicité et le coût sont également des paramètres importants à prendre en compte.

Aucune des techniques d'imagerie connues de l'art antérieur ne donne pleine satisfaction.

La tomographie par cohérence optique (OCT, de l'anglais « Optical Coherence Tomography ») à balayage est une technique basée sur l'interférométrie en lumière « blanche » (à large bande). Dans sa version dans le domaine temporel, un faisceau de lumière blanche est divisé en deux parties, l'une focalisée sur le tissu à étudier et l'autre sur un miroir de référence. La lumière réfléchie (rétrodiffusée) par l'objet observé est combinée avec celle réfléchie par le miroir de référence et détectée par un photodétecteur. Une interférence se produit seulement lorsque la différence de chemin optique est tout au plus de l'ordre de la longueur de cohérence du rayonnement ; en modifiant la longueur optique du bras de référence de l'interféromètre on accède à des profondeurs différentes dans l'objet. Une image à 2 voire 3 dimensions peut être construite grâce à l'interférométrie (qui permet l'acquisition selon la dimension axiale, c'est-à-dire la profondeur) et au balayage (qui permet l'acquisition selon une ou deux dimensions latérales). Dans l'OCT à balayage dans le domaine fréquentiel, le bras de référence a une longueur optique fixe et le signal interférométrique est analysé spectralement.

Voir à ce propos l'article de A. F. Fercher « Optical coherence tomography - principles and applications », Reports on Progress in Physics 66 (2003) 239 - 303. En pratique, l'OCT permet difficilement d'obtenir des résolutions latérales meilleures d'environ quelques micromètres.

Une technique plus récente, l'OCT plein champ, utilise un capteur bidimensionnel d'images pour détecter les signaux interférométriques. Cette technique, couplée avec l'utilisation d'une source de lumière de faible cohérence temporelle et spatiale telle qu'une lampe halogène, permet d'améliorer sensiblement la résolution spatiale - aussi bien latérale qu'en profondeur (axiale) - par rapport à l'OCT à balayage. Toutefois cette technique est peu adaptée aux applications dans lesquelles l'objet est susceptible de bouger (en particulier pour les applications *in vivo*), entraînant alors un brouillage des signaux interférométriques. Par ailleurs, elle fournit des coupes « en face » (parallèles à la surface de l'objet observé), alors que des coupes verticales sont souvent plus utiles. En outre, sa profondeur de pénétration est moindre qu'en OCT à balayage. Cette technique est décrite, par exemple, dans le document EP1364181 et dans l'article de A. Dubois, K. Grieve, G. Moneron, R. Lecaque, L. Vabre, et A.C. Boccara « Ultrahigh-resolution full-field optical coherence tomography », Applied Optics 43, p. 2874 (2004).

L'article de S. Kim et al. « Simultaneous measurement of refractive index and thickness by combining low-coherence interferometry and confocal optics », Optics Express, Vol. 16, No. 8, 5516 (14 avril 2008) décrit un procédé qui combine optique confocale et interférométrie à faible longueur de cohérence pour caractériser un échantillon en déterminant son épaisseur et son indice de réfraction. Il ne s'agit pas d'un procédé d'imagerie, encore moins de tomographie.

La microscopie confocale utilise un filtrage spatial pour sélectionner la lumière provenant d'une petite région de l'objet observé ; une image bi- ou tridimensionnelle peut alors être reconstituée par balayage. Le document EP 2 447 754 décrit un dispositif et un procédé de microscopie confocale chromatique à fentes. Ce système nécessite :

- un éclairage en lumière polarisée
- un objectif présentant de fortes aberrations chromatiques ; et
- un spectromètre pour mesurer le spectre de la lumière à la sortie du microscope, cette mesure permettant d'accéder à la profondeur sondée dans l'objet.

Dans ce dispositif, le rôle des fentes est de générer des lignes spectrales (leur largeur définissant la résolution spectrale et donc la résolution spatiale selon la profondeur dans l'objet). Elles n'ont pas un rôle de filtrage confocal.

Le document EP 1 586 931 décrit un autre dispositif et procédé de microscopie confocale à fente, qui simplifie le processus de reconstruction d'images en permettant l'acquisition simultanée de plusieurs pixels disposés suivant une ligne. La microscopie confocale, utilisée sans marqueurs fluorescents, offre une profondeur de pénétration sensiblement moindre qu'en OCT à balayage et en OCT plein champ.

L'article de Yu Chen et al. « High-resolution line-scanning optical coherence microscopy », Optics Letters Vol. 32, No. 14, 15 juillet 2007, pages 1971 - 1973 décrit un appareil et un procédé combinant microscopie confocale à fente et OCT à balayage permettant d'obtenir des coupes « en face » d'un échantillon avec une sensibilité plus élevée qu'en OCT plein champ. La résolution axiale atteinte est d'environ 3 $\mu$m et la résolution latérale d'environ 2 $\mu$m, ces résultats étant obtenus en utilisant comme source de lumière un laser à impulsions femtosecondes très couteux.

Les techniques de microscopie non linéaire (microscopie à deux photons, par génération d'harmonique...) présentent des performances - en termes de profondeur de pénétration, résolution spatiale et cadence d'acquisition - comparables à celles de l'OCT plein champ, mais au prix d'un coût plus élevé et de temps d'acquisition généralement plus longs.

Le coût et la complexité de mise en œuvre sont également parmi les principaux inconvénients des techniques d'imagerie non optiques, telles que la microtomographie X (qui présente également une faible cadence d'acquisition) et l'imagerie par résonance magnétique IRM (dont la résolution spatiale est médiocre comparée aux méthodes optiques).

L'invention vise à surmonter au moins certains des inconvénients précités de l'art antérieur. Plus particulièrement elle vise à procurer une technique de visualisation de la structure interne d'objets semi-transparents tels que des tissus biologiques permettant d'obtenir des coupes verticales (orthogonales à la surface de l'objet) à une cadence élevée (plusieurs coupes par seconde), avec une haute résolution spatiale (de l'ordre de 1 $\mu$m, aussi bien axialement que latéralement) et une profondeur de pénétration satisfaisante (de l'ordre du millimètre). L'invention vise également à procurer une technique adaptée aux applications *in vivo* et *in situ.*

Un objet de l'invention permettant d'atteindre ce but est un appareil de tomographie optique comprenant : une source de lumière polychromatique ; un capteur optique monodimensionnel ; un microscope interférométrique comprenant : un premier bras, dit de référence, à l'extrémité duquel est agencé un miroir dit de référence ; un second bras, dit objet ; un séparateur de faisceau couplant lesdits premier et second bras à ladite source de lumière polychromatique et audit capteur ; et au moins un objectif, ledit miroir de référence étant agencé en correspondance d'un plan de mise au point dudit objectif ou d'un dit objectif placé dans le bras de référence ; un système de filtrage spatial confocal monodimensionnel, coopérant avec ladite source de lumière polychromatique pour éclairer un objet à observer, agencé à l'extrémité dudit bras objet, selon une ligne, dite ligne d'observation, située dans ledit plan de mise au point dudit objectif ou d'un dit objectif placé dans le bras objet, ledit système de filtrage spatial confocal monodimensionnel étant également agencé pour sélectionner la lumière rétrodiffusée par ledit objet et provenant de ladite ligne d'observation, et pour former une image monodimensionnelle de ladite ligne sur ledit capteur ; caractérisé en ce qu'il comprend également : un système d'actionnement configuré pour déplacer ladite ligne d'observation parallèlement à un axe optique dudit objectif ou d'un dit objectif placé dans le bras objet de façon à réaliser un balayage unidirectionnel dudit objet, tout en maintenant une différence de chemin optique nulle entre, d'une part, un premier trajet allant dudit séparateur de faisceau audit miroir de référence et retour en parcourant ledit bras de référence et, d'autre part, un second trajet allant dudit séparateur de faisceau à ladite ligne d'observation et retour en parcourant ledit bras objet ; et un processeur programmé ou configuré pour reconstituer une image bidimensionnelle d'une section dudit objet à observer, orientée parallèlement audit axe optique dudit objectif ou d'un dit objectif placé dans le bras objet, à partir d'une pluralité d'images interférométriques monodimensionnelles acquises par ledit capteur en correspondance de positions différentes de ladite ligne d'observation au cours dudit balayage unidirectionnel.

Selon différents modes de réalisation d'un tel appareil :

- Ledit système d'actionnement peut être configuré pour provoquer un déplacement relatif, parallèle audit axe optique dudit objectif, ou d'un dit objectif placé dans le bras objet, dudit objet à observer par rapport audit microscope interférométrique, sans modifier les longueurs optiques dudit bras de référence et dudit bras objet.

- Ledit système d'actionnement peut être configuré pour déplacer l'objectif dans le plan de mise au point duquel se trouve ladite ligne d'observation et pour modifier la longueur optique dudit bras de référence de manière à maintenir nulle la différence de chemin optique entre ledit premier trajet et ledit second trajet.

- L'appareil peut comprendre également un dispositif de compensation de la dispersion agencé sur au moins un parmi ledit bras objet et ledit bras de référence, ledit système d'actionnement étant configuré pour agir également sur ledit dispositif de compensation de la dispersion au cours dudit balayage unidirectionnel.

- Ledit microscope interférométrique peut être un microscope de Linnik, comprenant un premier objectif agencé sur ledit bras de référence et un deuxième objectif agencé sur ledit bras objet, lesdits bras de référence et objet étant disjoints. En variante, il peut être choisi parmi un microscope de Michelson et un microscope de Mirau, comprenant un objectif unique.

Un autre objet de l'invention est un procédé de tomographie optique comprenant les étapes suivantes :

a) procurer une source de lumière polychromatique ;
b) utiliser un séparateur de faisceau pour diriger une première fraction de lumière émise par ladite source suivant un premier trajet, dit trajet de référence, et une seconde fraction de lumière émise par ladite source suivant un second trajet, dit trajet objet ;
c) utiliser un objectif coopérant avec un système de filtrage spatial confocal monodimensionnel pour focaliser ladite seconde fraction de lumière de façon à éclairer un objet semi-transparent à observer selon une ligne, dite ligne d'observation, située dans un plan de mise au point dudit objectif, et pour collecter la lumière rétrodiffusée par ledit objet ainsi éclairé ;
d) utiliser ledit objectif, ou un autre objectif, pour focaliser ladite première fraction de lumière sur un miroir de référence agencé sur ledit trajet de référence, et pour collecter la lumière réfléchie par ledit miroir ;
e) utiliser ledit séparateur de faisceau pour combiner la lumière rétrodiffusée par ledit objet avec la lumière réfléchie par ledit miroir et la diriger vers un capteur optique monodimensionnel ;
f) utiliser ledit système de filtrage spatial confocal monodimensionnel pour sélectionner la lumière provenant de ladite ligne d'observation, et pour en former une image monodimensionnelle sur ledit capteur ;
g) utiliser un système d'actionnement pour déplacer ladite ligne d'observation parallèlement à un axe optique dudit objectif de façon à réaliser un balayage unidirectionnel d'un objet à observer sur ledit trajet objet, tout en maintenant une différence de chemin optique nulle entre ledit trajet de référence et ledit trajet objet ; et
h) utiliser un processeur pour reconstituer une image bidimensionnelle d'une section dudit objet à observer, orientée parallèlement audit axe optique, à partir d'une pluralité d'images interférométriques monodimensionnelles acquises par ledit capteur en correspondance de positions différentes de ladite ligne d'observation au cours dudit balayage unidirectionnel.

Selon différents modes de réalisation d'un tel procédé :

- Ladite étape g) peut être mise en œuvre en provoquant un déplacement relatif, parallèle audit axe optique, dudit objet à observer par rapport audit microscope interférométrique sans modifier les longueurs optiques dudit bras de référence et dudit bras objet.
- Alternativement, ladite étape g) peut être mise en œuvre en déplaçant l'objectif dans un plan focal duquel se trouve ladite ligne d'observation et en modifiant la longueur optique dudit bras de référence de manière à maintenir nulle la différence de chemin optique entre ledit premier trajet et ledit second trajet.
- Le procédé peut comprendre également une étape i) de compensation des modifications de la dispersion induites par le déplacement de la ligne d'observation à l'intérieur dudit objet à observer au cours dudit balayage unidirectionnel.

D'autres caractéristiques, détails et avantages de l'invention ressortiront à la lecture de la description faite en référence aux dessins annexés donnés à titre d'exemple et qui représentent, respectivement :

- La figure 1A, un schéma de principe d'un appareil de tomographie optique selon un mode de réalisation de l'invention, basé sur un microscope interférométrique de Linnik ;
- La figure 1B, un détail d'un appareil de tomographie optique selon une variante dudit mode de réalisation de l'invention ;
- Les figures 2A, 2B, d'autres modes possibles de réalisation de l'invention basés sur les configurations de microscopes interférométriques de Michelson et de Mirau ;
- Les figures 3A et 3B, le principe du filtrage confocal à fente ;
- Les figures 4A et 4B, respectivement, une image obtenue en utilisant un appareil de tomographie par cohérence optique selon l'art antérieur et une image du même objet obtenue en utilisant un appareil de tomographie optique selon l'invention ;
- Les figures 5A à 5G, différents modes de réalisation de l'invention basés sur des microscopes interférométriques de Michelson et de Mirau et utilisant un milieu d'immersion ; et
- La figure 6, un autre mode de réalisation de l'invention basé sur un microscope interférométrique de Linnik et utilisant un milieu d'immersion.

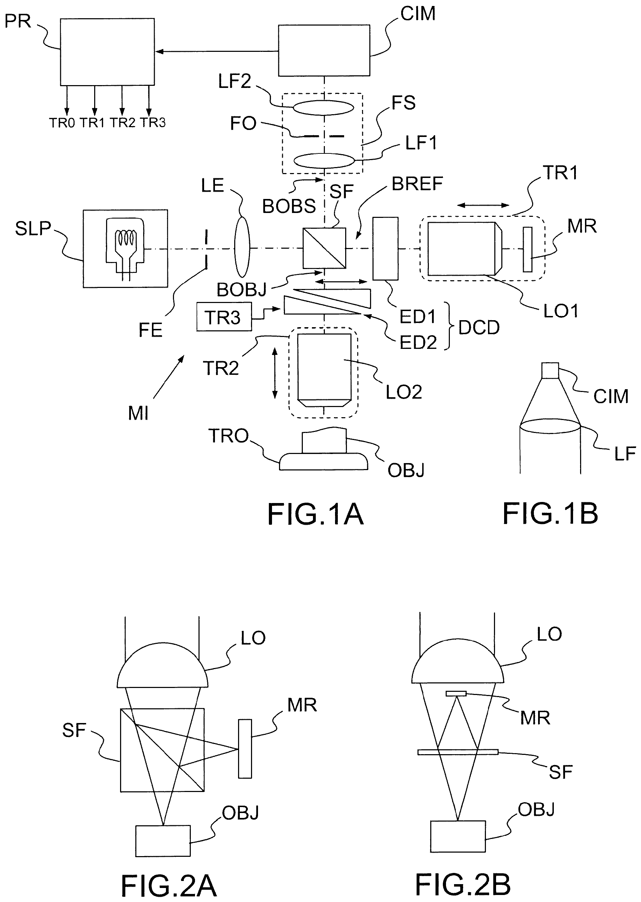

La figure 1A illustre un appareil de tomographie optique selon un mode de réalisation de l'invention. Cet appareil est essentiellement constitué par un microscope interférométrique « de Linnik » modifié par l'ajout de moyens de filtrage spatial (FE, FS), d'un système de compensation (DCD) des écarts de dispersion entre les deux bras de l'interféromètre, d'un capteur optique monodimensionnel (CIM) et d'un processeur programmé ou configuré de manière opportune (PR).
Cet appareil comprend une source de lumière polychromatique SLP. Cette dernière est représentée schématiquement par une ampoule à incandescence, mais il pourra s'agir de préférence d'une source de luminance plus élevée, telle qu'une diode électroluminescente ou une association de diodes électroluminescentes, d'une diode superluminescente ou une association de diodes superluminescentes, d'une lampe à filament halogène, d'une lampe à arc, voire d'une source laser où à base de laser (source par génération de « supercontinuum » par exemple). Dans tous les cas, sa largeur spectrale (à mi-hauteur) sera de préférence supérieure ou égale à 100 nm ; plus cette largeur spectrale est importante, meilleure pourra être la résolution axiale de l'appareil ; la longueur d'onde de centre bande peut être visible ou se situer dans le proche infrarouge ; dans les applications biologiques et médicales on préfère généralement le proche infrarouge, entre 600 nm et 1500 nm typiquement. La source peut être polarisée ou non polarisée, spatialement cohérente ou incohérente. Les sources spatialement cohérentes (de types lasers ou diodes superluminescentes) peuvent être avantageuses en raison de leur plus grande luminance, mais elles peuvent introduire du « bruit » de cohérence : phénomènes d'interférences parasites entraînant une réduction de l'amplitude relative du signal interférométrique utile et un manque d'uniformité de l'éclairage. En outre, l'utilisation de sources spatialement cohérentes augmente sensiblement le coût global de l'appareil.

Une fente FE et une lentille LE forment un système optique d'éclairage coopérant avec la source SLP et le microscope interférométrique pour éclairer un objet OBJ à observer suivant une ligne. Si la source de lumière polychromatique est spatialement cohérente, la fente FE peut être remplacée par une optique de mise en forme de faisceau, incorporant par exemple une lentille cylindrique, convergente ou divergente, afin d'éclairer l'objet selon une ligne ayant une largeur de l'ordre du micromètre (plus précisément, la largeur de la ligne est de l'ordre de grandeur de la résolution latérale du système d'imagerie).

Le faisceau d'éclairage formé par la lentille LE est dirigé vers un séparateur de faisceau - en l'espèce, un cube séparateur - SF. Ce dernier dirige une première portion du faisceau incident le long d'un premier bras du microscope interférométrique, dit « bras de référence », BREF et une seconde portion du faisceau incident suivant un second bras BOBJ, dit « bras objet ». Un premier objectif de microscope LO1 et un miroir dit « de référence » MR sont agencés sur le bras de référence ; l'objectif focalise la lumière sur le miroir, puis recueille la lumière réfléchie par ce dernier et la redirige - en sens inverse - suivant le bras, ou trajet, de référence. Un second objectif de microscope LO2, de longueur focale identique à celle dudit premier objectif LO1, est agencé sur le bras objet ; l'objectif focalise la lumière sur l'objet OBJ à observer, puis recueille la lumière rétrodiffusée par ce dernier et la redirige - en sens inverse - suivant le bras, ou trajet, objet. Typiquement les objectifs ont une ouverture numérique comprise entre 0,1 et 1,0 (contrairement à l'OCT à balayage traditionnelle, il n'y a pas ici de contrainte de profondeur de champ qui limiterait l'ouverture numérique à employer). Il est intéressant de noter que ces objectifs peuvent être dans l'air ou à immersion ; par contre, dans le cas de l'OCT plein champ on utilise des objectifs à immersion, ce qui peut être contraignant dans certaines applications.

Le séparateur de faisceau SF recombine les faisceaux lumineux en provenance des deux objectifs, leur permettant d'interférer, et les redirige le long d'un bras dit « d'observation » BOBS.

Le contraste des franges d'interférence est maximal lorsque les deux faisceaux qui interfèrent présentent une même intensité ; par conséquent il peut être avantageux d'utiliser un miroir de référence faiblement réfléchissant ou de prévoir un atténuateur sur le trajet de référence.

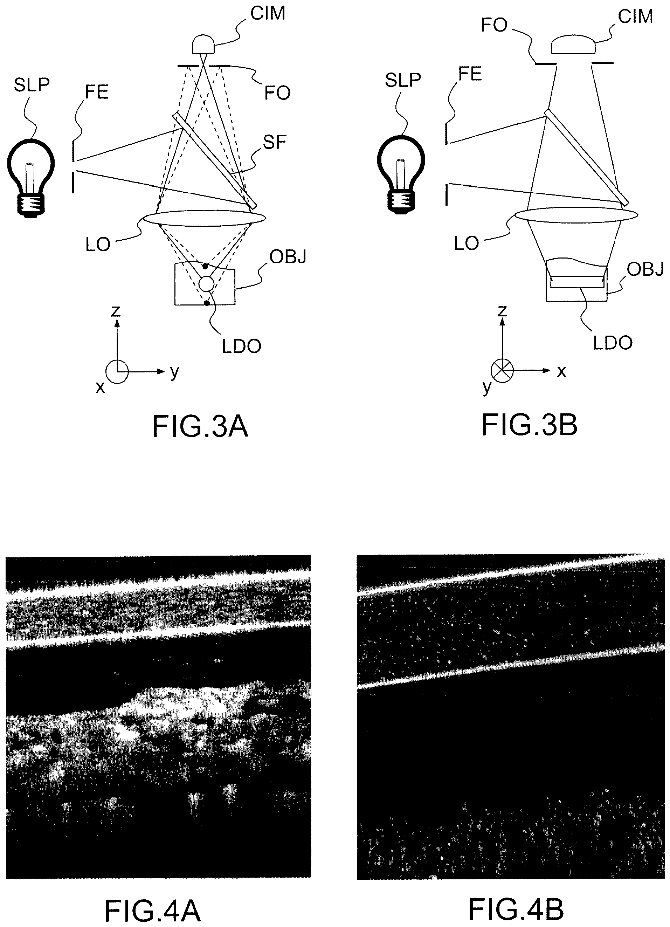

Un filtre spatial monodimensionnel FS est agencé sur le bras d'observation. Dans le mode de réalisation de la figure 1A il s'agit d'un filtre confocal, comprenant deux lentilles LF1, LF2. Une fente FO est placée dans le plan focal arrière de la lentille LF1. La lentille LF2 forme une image de la fente FO sur le capteur optique monodimensionnel CIM. La fente FO est optiquement conjuguée à la fente FE associée à la source SLP ; autrement dit, le microscope interférométrique forme une image de la fente FE en correspondance de la fente FO et inversement. Comme cela sera expliqué plus loin, en référence aux figures 3A et 3B, il s'agit là d'une configuration « confocale » ; en effet, la source SLP, la fente FE, la lentille LE, le séparateur SF, l'objectif LO2, le filtre spatial SF forment un microscope confocal à fente.

Le capteur optique monodimensionnel CIM (caméra linéaire), constitué par une rangée unique de pixels (carrés ou rectangulaires), ou de quelques (typiquement pas plus de 10 ou 20, voire 100 au maximum) rangées de pixels, détecte la lumière en sortie du filtre. Il est également possible d'utiliser comme capteur CIM une seule rangée de pixels, ou la combinaison de quelques (le plus souvent jusqu'à 10 ou 20) rangées de pixels, adjacentes ou proches, d'un capteur d'image matriciel.

En variante (illustrée sur la figure 1B) le filtrage spatial peut être réalisé sans le système de filtrage FS, en positionnant le capteur optique monodimensionnel CIM dans le plan focal d'une lentille unique LF.

En outre, si une fente FO est présente du côté du détecteur (ou que le détecteur fait lui-même office de fente comme expliqué précédemment, figure 1B), il est possible d'omettre la fente d'éclairage FE (ou le système de mise en forme du faisceau la remplaçant), au prix d'une baisse éventuelle de la sensibilité de détection.

L'appareil comprend également un système d'actionnement constitué par une pluralité d'étages de translation - TR1, TR2, TR3 et TRO - et par un processeur PR les pilotant. Tous ces étages de translation ne doivent pas nécessairement être présents en même temps ; en particulier, si TRO est présent, TR1 et TR2 peuvent être omis, et inversement si TR1 et TR2 sont présents, TRO peut être omis.

L'ensemble miroir de référence MR et objectif LO1 est déplacé axialement au moyen d'un premier étage de translation TR1 dudit système d'actionnement ; par conséquent, l'objectif LO2 doit également être déplacé, au moyen d'un étage de translation respectif TR2, faisant aussi partie dudit système d'actionnement. Lorsqu'on descend (c'est-à-dire qu'on translate en direction de l'objet) l'objectif LO2 d'une distance « e », on déplace le miroir de référence MR et l'objectif LO1 de

$$\left( n_{im} - 1 - \frac{n_{objet}^2}{n_{im}} \right) e,$$ $n_{im}$ étant l'indice de réfraction du milieu d'immersion des objectifs - gel, liquide ou air ($n_{im} = 1$) - et $n_{objet}$ étant l'indice de réfraction de l'objet.

En variante, il serait possible de faire varier la distance axiale entre le seul objet OBJ et le microscope interférométrique, en laissant stationnaires les différents éléments du microscope interférométrique. Pour cela, on peut déplacer tout le microscope interférométrique (système de déplacement non représenté) ou l'objet OBJ au moyen de l'étage de translation TRO ; ce dernier cas est envisageable en particulier avec des objectifs à immersion.

Dans tous les cas, cela a pour effet de modifier la profondeur à laquelle est sondé l'objet OBJ : une ligne d'observation LDO, située dans le plan focal (plus généralement, dans le plan de mise au point) de l'objectif LO2, réalise un balayage dudit objet « en profondeur », c'est-à-dire dans la direction de l'axe optique dudit objectif. Le système d'actionnement doit à la fois déplacer cette ligne d'observation et assurer que la différence de chemin optique entre le trajet de référence et le trajet objet (jusqu'à la ligne d'observation, qui est considérée comme constituant l'extrémité du bras objet) reste nulle ou au plus inférieure à la longueur de cohérence de la source de lumière polychromatique et à la profondeur de champ de l'objectif. Ce balayage modifie l'épaisseur d'objet traversé par la lumière se propageant le long du bras objet BOBJ, et donc la dispersion qu'elle subit. Un dispositif DCD est prévu pour compenser cette modification de la dispersion. Le dispositif DCD comprend un élément à dispersion constante ED1 - par exemple un bloc de verre, matériau qui a une dispersion proche de celle de l'objet OBJ - agencé dans l'un des bras de l'interféromètre et un élément à dispersion variable ED2 disposé dans l'autre bras de l'interféromètre. L'élément ED2 est constitué de deux prismes agencés face à face ; en déplaçant un des prismes par rapport à l'autre, on modifie l'épaisseur de verre traversé, et donc le trajet optique dans ce bras. On peut également utiliser une lame de verre inclinée par rapport au trajet optique ; l'épaisseur de verre traversée est modifiée en agissant sur l'angle d'inclinaison. D'autres systèmes peuvent être envisagés ; l'idée générale est qu'il faut faire varier l'épaisseur optique dans l'un des bras afin d'égaler la dispersion dans les 2 bras de l'interféromètre (ou du moins de réduire l'écart) quelle que soit la profondeur d'imagerie.

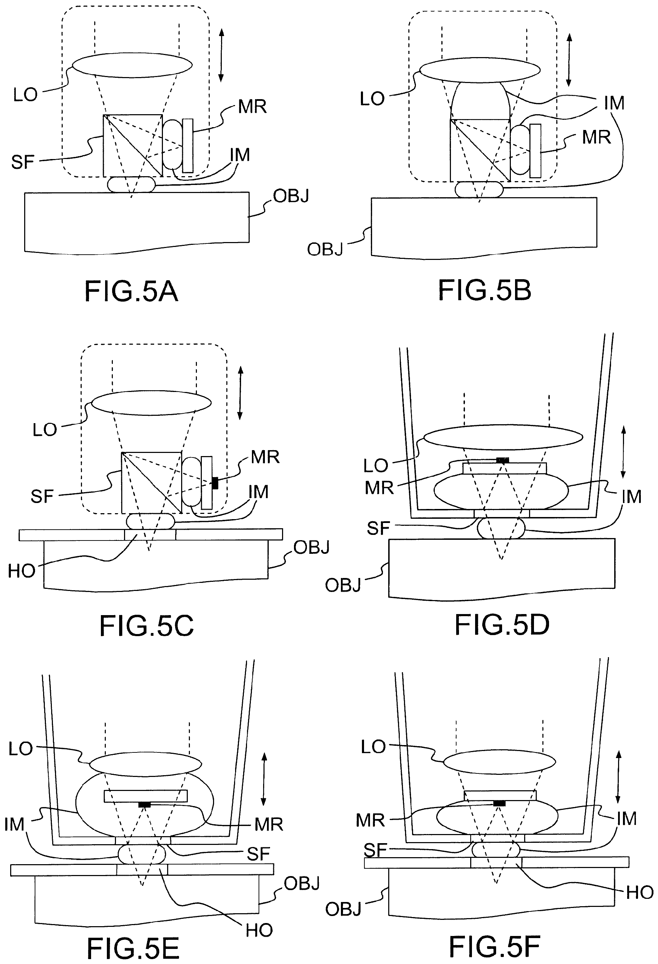

Dans d'autres modes de réalisation, le dispositif de compensation de la dispersion peut être constitué plus simplement par un milieu d'immersion (typiquement une goutte de liquide dont l'indice de réfraction est proche de celui de l'objet) dont l'épaisseur dans le bras objet se réduit au fur et à mesure que l'objectif se rapproche de l'objet à observer (voir les figures 5A à 5F). La diminution de l'épaisseur du milieu d'immersion est compensée par l'augmentation de l'épaisseur traversée dans l'objet : ainsi la dispersion dans le bras objet reste constante, égale à celle dans le bras de référence.

Dans le mode de réalisation de la figure 1, le dispositif DCD est actionné par un troisième étage de translation TR3, faisant également partie dudit système d'actionnement. Cela permet de réaliser une compensation dynamique de la dispersion, synchronisée avec les autres déplacements.

En variante, on peut utiliser une épaisseur variable de matériau dispersif transparent, tel que le verre, placé dans l'un des bras de l'interféromètre pour modifier à la fois le chemin optique et la dispersion. Cette épaisseur variable peut être réalisée, par exemple, grâce à un double prisme, comme le dispositif DCD de la figure 1, ou une simple lame orientable. Dans ce cas, il peut ne pas être nécessaire de déplacer l'ensemble objectif LO1 et miroir MR.

Le détecteur CIM acquiert des images-lignes en correspondance d'une pluralité de positions différentes de la ligne imagée dans l'objet. Cette pile d'images-lignes peut être traitée numériquement pour obtenir une image d'une coupe verticale de l'objet.

Une approche simple consiste à utiliser la méthode dite d'interférométrie à décalage de phase, consistant à combiner numériquement plusieurs images-lignes déphasées. Par exemple, on peut combiner quatre images-lignes correspondant à des positions de la ligne d'observation espacées de $\lambda/8$ dans la direction axiale, $\lambda$ étant la longueur d'onde centrale de la lumière d'éclairage dans l'objet. Cela correspond à un déphasage de $\pi/2$ entre deux images adjacentes. Si l'on indique par $E_1$, $E_2$, $E_3$, $E_4$ ces images, $(E_1 - E_3)^2 + (E_2 - E_4)^2$ correspond à l'amplitude du signal d'interférence - c'est-à-dire à l'amplitude de l'image reconstituée - et $\frac{(E_1 - E_3)}{(E_2 - E_4)}$ correspond à la phase du signal d'interférence. Cette phase peut fournir d'autres informations que des informations structurelles et tomographiques sur l'objet. Il faut noter qu'il n'y a pas de contradiction entre la notion de décalage de phase, ou déphasage, et le fait, mentionné plus haut, que la ligne d'observation correspond toujours à une différence de chemin optique entre les bras objet et de référence égale à zéro. En effet, une structure quelconque de l'objet, susceptible de rétrodiffuser la lumière, n'est pas observée uniquement lorsqu'elle coïncide avec la ligne d'observation, mais également avant et après (car la « porte » de cohérence et celle introduite par le filtrage confocal ont une largeur supérieure à $\lambda$). Il y a donc bien un décalage de phase entre les contributions de cette structure aux images acquises successivement au cours du balayage axial.

En variante, la pile d'images-lignes peut être traitée par analyse de Fourier afin d'extraire l'enveloppe des franges d'interférence (l'amplitude du signal d'interférence) et éliminer la partie non modulée du signal (signal non interférométrique).

Il convient de souligner ici que, conformément à l'invention, le balayage unidirectionnel de l'objet sur toute la profondeur imagée afin de produire une image en coupe axiale permet également d'acquérir en même temps un signal interférométrique (bien entendu, un deuxième balayage unidirectionnel peut ensuite être réalisé dans le sens opposé). Par contre, aussi bien dans la technique précitée de Yu Chen et al. qu'en OCT plein champ, il n'y a pas de balayage de la profondeur de l'objet pour acquérir le signal interférométrique. Ce dernier est acquis grâce à un déphasage variable et périodique réalisé par déplacement du miroir de référence sur une plage totale inférieure à 1 micromètre typiquement.

Une image tridimensionnelle de l'objet peut être obtenue en juxtaposant des images en coupes adjacentes. Cela nécessite un balayage dans une direction perpendiculaire aussi bien à la ligne d'acquisition qu'à l'axe optique de l'objectif LO2. Ce balayage peut être obtenu en déplaçant l'objet (ou, de manière équivalente, le microscope interférométrique, ou la ligne d'illumination) au moyen d'un étage de translation latérale.

Un même processeur PR peut piloter les actionneurs TR1, TR2, TR3 et, le cas échéant, TRO (qui peuvent ne pas être, ou ne pas être uniquement, des étages de translation) et traiter les piles d'images-lignes acquises par le capteur CIM, ces tâches étant interdépendantes. Le processeur PR peut être un dispositif dédié, comprenant un ou plusieurs microprocesseurs, ou un ordinateur équipé de cartes d'interface opportunes. En variante, deux processeurs distincts peuvent être utilisés pour le pilotage du système d'actionnement et pour la reconstruction des images.

L'invention a été décrite en référence à un mode de réalisation particulier, basé sur un microscope interférométrique de Linnik. Cependant, d'autres types de microscopes interférométriques existent et conviennent à la mise en œuvre de l'invention. On peut citer par exemple les microscopes de Michelson (figure 2A) et de Mirau (figure 2B) comprenant un seul objectif LO avec un miroir de référence MR et un séparateur de faisceau solidaires dudit objectif et alignés le long de son axe optique. Ces montages sont plus simples et plus compacts que celui de Linnik, mais l'introduction d'un dispositif de compensation ajustable de la dispersion, ainsi que le déplacement éventuel d'élément(s) de l'interféromètre, sont plus difficiles. Des exemples de réalisations basées sur les configurations de Michelson et de Mirau utilisant un milieu d'immersion IM pour compenser l'écart de dispersion entre les deux bras de l'interféromètre sans besoin de parties en mouvement à l'intérieur de l'interféromètre sont illustrés sur les figures 5A (configuration de Michelson avec objectif à air), 5B (configuration de Michelson avec objectif à immersion), 5C (configuration de Michelson avec hublot d'observation HO et objectif à air - à noter que le hublot peut être remplacé par un trou et/ou l'objectif être à immersion; si le hublot est présent, une lame transparente de la même épaisseur doit être prévue dans le bras de référence), 5D (configuration de Mirau avec objectif à air), 5E (configuration de Mirau avec objectif à immersion et hublot), 5F (configuration de Mirau avec objectif à air et hublot et 5G (configuration de Mirau avec objectif à immersion, sans hublot).

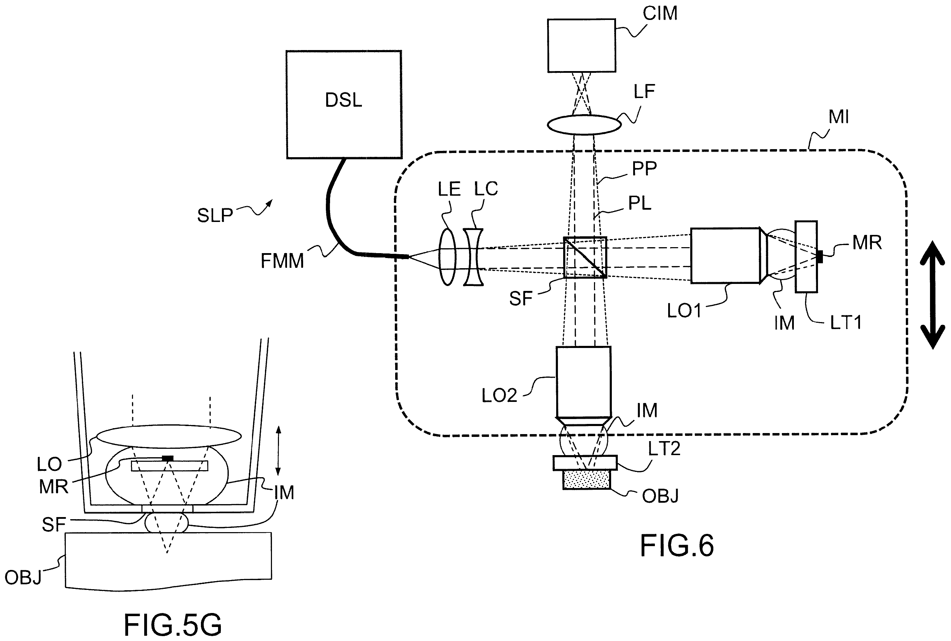

Dans les dispositifs des figures 5D à 5G le miroir de référence est formé par une lame transparente présentant une petite zone réflectrice en son centre, réalisée par exemple par un dépôt métallique ou diélectrique ou induite par l'indice de réfraction de la lame elle-même. Dans les modes de réalisation des figures 5C, 5D et 5G, la zone réflectrice est formée sur la face arrière de la lame transparente, qui sert à compenser la dispersion introduite, dans le bras objet, par le hublot (figure 5C) ou le séparateur de faisceau (5D, 5G). Sinon, elle est formée sur la face avant de la lame. En outre, dans les modes de réalisation des figures 5D à 5G le séparateur de faisceau est constitué par une lame présentant un coefficient de réflexion adapté (entre 10% et 50% typiquement) sur une de ses faces ; la séparation se fait sur la face avant de cette lame dans le cas des figures 5D et 5G, et sur la face arrière dans le cas des figures 5E et 5F. Les faces « avant » et « arrière » sont définies par rapport à la direction de propagation du faisceau lumineux incident sur la lame.

La figure 6 illustre un autre mode de réalisation de l'invention dans lequel :

- Le microscope interférentiel MI est du type de Linnik ;
- Les objectifs LO1, LO2 sont du type à immersion. Ainsi, la compensation de dispersion est réalisée par deux gouttes d'un milieu d'immersion IM. Chacune de ces gouttes est enserrée entre un objectif (LO1, LO2) et une lame transparente (LT1, LT2). Le miroir de référence est réalisé par la face arrière (opposée à l'objectif LO1) de la lame LT1 (face éventuellement traitée pour ajuster son coefficient de réflexion), tandis que l'objet OBJ est appliqué contre la face arrière de la lame LT2.
- La source de lumière polychromatique SLP est spatialement cohérente. Elle comprend une source polychromatique primaire, qui est en particulier une diode superluminescente DSL présentant une longueur de cohérence temporelle de l'ordre de 1 à 20 $\mu$m, ainsi qu'une fibre optique monomode FMM (optionnelle). La lumière générée par la diode superluminescente est injectée dans une extrémité dite d'entrée de la fibre FMM, sort de l'extrémité opposée (de sortie) de ladite fibre et est collimatée par une lentille LE.

- La cohérence spatiale de l'éclairage permet de réaliser un filtrage confocal particulièrement simple, ne nécessitant pas de fente et comportant seulement une lentille cylindrique LC divergente du côté de la source de lumière (en fait, de l'extrémité de sortie de la fibre optique) et une lentille sphérique LF agencée devant le capteur CIM, de type monodimensionnel. La lentille cylindrique LC crée un astigmatisme en rendant le faisceau lumineux divergent dans le plan de la figure, mais pas dans un plan perpendiculaire (la ligne pointillée PP représente une section des faisceaux lumineux dans le plan de la figure, tandis que la ligne tiretée PL représente une section des faisceaux lumineux dans un plan perpendiculaire à la figure). Il en résulte un éclairage de l'objet et du miroir de référence en forme de ligne orientée selon le plan de la figure ; la lentille LF crée une image de cette ligne sur le capteur optique monodimensionnel ; la lumière ne provenant pas de la ligne d'observation atteint la ligne unique de pixels du capteur avec une intensité atténuée, réalisant ainsi un filtrage confocal monodimensionnel. En variante, on pourrait utiliser une lentille cylindrique convergente pour rendre également le système astigmatique.

- Le balayage axial est réalisé en déplaçant le microscope interférométrique dans son ensemble - y compris l'extrémité de sortie de la fibre FMM et le système optique astigmatique formé par les lentilles LE et LC - par rapport à l'objet OBJ et à la lame transparente LT2. Le capteur optique CIM et la lentille associée LF peuvent indifféremment se déplacer avec le microscope interférométrique ou pas (la deuxième option est illustrée sur la figure) ; il est cependant nécessaire que le capteur CIM reste dans le plan focal (ou plus généralement le plan de mise au point) de la lentille LF.

Les figures 3A et 3B rappellent le principe de fonctionnement d'un microscope confocal à fente. Dans un plan perpendiculaire à la fente de filtrage spatial FO (plan zy sur la figure 3A), la fente FO laisse passer la lumière provenant de la région de l'objet où la fente FE est focalisée par l'objectif LO (faisceau représenté en trait continu) et atténue la lumière provenant d'autres régions de l'objet (faisceaux en lignes pointillées par exemple). Dans un plan parallèle à la fente (plan zx sur la figure 3A), un tel filtrage ne se produit pas. Le résultat est que l'on définit une « ligne d'observation » LDO dans le plan de mise au point de l'objectif et orientée comme les fentes FE et FO. Les plans de mise au point sont les plans, perpendiculaires à l'axe optique, où sont formées les images de la fente d'entrée FE, si elle est présente. Plus généralement, le plan de mise au point du bras objet est celui où se forme la ligne d'observation LDO; son plan conjugué est le plan de mise au point dans le bras d'observation; et le plan de mise au point du bras de référence est celui où une ligne d'éclairage se forme dans ce bras. Si le faisceau lumineux d'entrée est collimaté dans le plan orthogonal à la ligne d'observation ou d'éclairage, comme dans les figures 1A, 1B, les plans de mise au point correspondent avec les plan focaux du ou des objectifs.

Un appareil selon l'invention comprend ainsi deux moyens permettant de sélectionner la lumière provenant d'une région déterminée de l'objet : le filtrage confocal et la « porte de cohérence » définie par l'interférométrie en lumière « blanche ». Ces moyens sont complémentaires : le filtrage confocal améliore les performances de l'imagerie interférométrique en réduisant le « fond » produit par la diffusion de la lumière et par des réflexions parasites. Une plus grande partie de la dynamique du capteur est ainsi utilisée pour détecter le signal interférométrique utile.

En effet, les tissus biologiques sont fortement diffusants ; le nombre de photons balistiques (n'ayant pas subi de diffusion autre qu'une seule rétro-diffusion) diminue exponentiellement avec la profondeur. Or, l'interférométrie - même à faible longueur de cohérence - ne permet pas de distinguer entre un photon balistique provenant de la région imagée dans l'objet et un photon provenant d'autres régions de l'objet et ayant parcouru un trajet optique de même longueur en raison des diffusions subies. Il en résulte un signal interférométrique parasite qui s'ajoute au signal interférométrique utile, créant des artefacts dans les images et limitant la profondeur d'imagerie accessible. Dans le dispositif de l'invention, le filtrage confocal élimine une grande partie des photons autres que les photons balistiques provenant de la zone imagée, supprimant ce fond ; certes, le filtrage confocal à fente n'est pas parfait car il n'agit que dans une dimension, mais la réduction de ce « bruit de fond » demeure significative.

Par rapport à un filtrage confocal seul, l'utilisation d'une détection interférométrique permet une considérable amplification du signal utile (dans le cas de la microscopie confocale « pure », c'est le faible rapport signal sur bruit qui limite la profondeur d'acquisition). Dans l'appareil de l'invention il y a donc synergie - et non simple juxtaposition - entre les deux principes mis en jeu : microscopie par interférométrie à faible longueur de cohérence et microscopie confocale à fente.

L'utilisation d'un filtrage confocal par un trou, au lieu d'un filtrage confocal à fente, ralentirait considérablement l'acquisition des images (un balayage supplémentaire serait nécessaire) sans apporter un avantage significatif en termes de résolution et/ou de profondeur d'imagerie accessible.

Le dispositif peut toutefois présenter des performances acceptables même si le filtrage confocal n'est pas efficace. Cette situation se présente lorsqu'on utilise des objectifs de profondeur de champ relativement grande (typiquement supérieure ou égale à 10 μm), et/ou des fentes en entrée et en sortie peu étroites et/ou un détecteur monodimensionnel ayant des pixels de grandes taille (rectangulaires par exemple) ou présentant plusieurs rangées de pixels. Dans ce cas, c'est la détection interférométrique à faible cohérence temporelle qui détermine les performances en termes de résolution axiale et de profondeur d'imagerie accessible. Mais l'intérêt de l'éclairage selon une ligne et la détection de cette ligne par un capteur monodimensionnel est encore présent, car cela permet toujours de produire des images en coupe verticale à grande vitesse.

La technique de l'invention est beaucoup mieux adaptée que l'OCT plein champ aux applications *in vivo*. Dans ces applications, l'« objet » est un organisme vivant (par exemple un patient) qui est susceptible de bouger au cours de l'acquisition du signal interférométrique, brouillant ce dernier. Conformément à l'invention, un signal interférométrique est acquis par ligne, en un temps très court, par exemple de l'ordre de 10$^{-4}$ seconde, ce qui permet de s'affranchir des problèmes de mouvement de l'objet. Par contre, en OCT plein champ, le signal interférométrique est acquis par combinaison de plusieurs images bidimensionnelles acquises par une caméra matricielle, ce qui est beaucoup plus long.

Par rapport à la technique précitée de Yu Chen et al., l'invention présente l'avantage de fournir directement des images de tranches verticales de l'objet observé, souvent plus utiles que des images « en face ».

Un prototype de l'invention a été réalisé, utilisant un éclairage par lampe halogène, des objectifs avec ouverture numérique de 0,15 dans l'air et une configuration de Linnik. Ce prototype a été testé en utilisant en tant qu'objet observé une carte de visualisation d'un faisceau infrarouge d'une épaisseur d'environ 500 μm, comprenant deux couches de plastique enfermant un plastique granuleux contenant des microstructures, une région non diffusante et une couche de fluorophores. Le même objet a été imagé en utilisant un appareil d'OCT à balayage du commerce (ThorLabs). La figure 5A montre l'image (tranche verticale) obtenue avec l'appareil du commerce, la figure 5B celle obtenue avec le prototype de l'invention. On peut noter à quel point la résolution est meilleure dans le cas de l'invention.

Il est intéressant de noter que, avec un dimensionnement approprié, un appareil selon l'invention peut présenter une résolution spatiale essentiellement « isotrope », de l'ordre de 1 μm aussi bien axialement que latéralement.

**Revendications**

1. Appareil de tomographie optique comprenant :

   - une source de lumière polychromatique (SLP) ;
   - un capteur optique monodimensionnel (CIM) ;
   - un microscope interférométrique (MI) comprenant : un premier bras (BREF), dit de référence, à l'extrémité duquel est agencé un miroir (MR) dit de référence ; un second bras (BOBJ), dit objet ; un séparateur de faisceau (SF) couplant lesdits premier et second bras à ladite source de lumière polychromatique et audit capteur ; et au moins un objectif (LO1, LO2, LO), ledit miroir de référence étant agencé dans un plan de mise au point, situé dans le bras de référence, dudit ou d'un dit objectif (LO, LO1) ;
   - un système de filtrage spatial confocal monodimensionnel (FE, FS, CIM), coopérant avec ladite source de lumière polychromatique pour éclairer un objet à observer (OBJ), agencé dans ledit bras objet (BOBJ), selon une ligne, dite ligne d'observation (LDO), gisant dans un plan de mise au point, situé dans le bras objet, dudit ou d'un dit objectif (LO, LO2), et pour former une image monodimensionnelle de ladite ligne d'observation sur ledit capteur ; **caractérisé en ce qu'**il comprend également :
   - un système d'actionnement (PR, TR1, TR2, TR3) configuré pour déplacer ladite ligne d'observation parallèlement à un axe optique s'étendant le long du bras objet dudit ou d'un dit objectif (LO, LO2) de façon à réaliser un balayage unidirectionnel dudit objet, tout en maintenant une différence de chemin optique nulle entre, d'une part, un premier trajet allant dudit séparateur de faisceau audit miroir de référence et retour en parcourant ledit bras de référence et, d'autre part, un second trajet allant dudit séparateur de faisceau à ladite ligne d'observation et retour en parcourant ledit bras objet ; et
   - un processeur (PR) programmé ou configuré pour reconstituer une image bidimensionnelle d'une section dudit objet à observer, orientée parallèlement audit axe optique s'étendant le long du bras objet dudit objectif (LO, LO2), à partir d'une pluralité d'images interférométriques monodimensionnelles acquises par ledit capteur en correspondance de positions différentes de ladite ligne d'observation au cours dudit balayage unidirectionnel.

2. Appareil selon la revendication 1 dans lequel ledit système de filtrage spatial confocal monodimensionnel est également agencé pour sélectionner la lumière rétrodiffusée par ledit objet et provenant de ladite ligne d'observation (LDO).

3. Appareil selon l'une des revendications précédentes dans lequel ledit système d'actionnement est configuré pour provoquer un déplacement relatif, parallèle audit axe optique dudit objectif (LO) ou d'un dit objectif placé dans le bras objet (LO2), dudit objet à observer par rapport audit microscope interférométrique, sans modifier les longueurs optiques dudit bras de référence et dudit bras objet.

4. Appareil selon l'une des revendications 1 ou 2 dans lequel ledit système d'actionnement est configuré pour déplacer l'objectif (LO2) dans le plan de mise au point duquel se trouve ladite ligne d'observation et pour modifier la longueur optique dudit bras de référence de manière à maintenir nulle la différence de chemin optique entre ledit premier trajet et ledit second trajet.

5. Appareil selon l'une des revendications précédentes comprenant également un dispositif de compensation de la dispersion (DCD, IM) agencé sur au moins un parmi ledit bras objet et ledit bras de référence, ledit système d'actionnement étant configuré pour agir également sur ledit dispositif de compensation de la dispersion au cours dudit balayage unidirectionnel.

6. Appareil selon l'une des revendications précédentes dans lequel ledit microscope interférométrique est un microscope de Linnik, comprenant un premier objectif (LO1) agencé sur ledit bras de référence et un deuxième objectif (LO2) agencé sur ledit bras objet, lesdits bras de référence et objet étant disjoints.

7. Appareil selon la revendication 3 dans lequel ledit microscope interférométrique est un microscope de Linnik, comprenant un premier objectif à immersion (LO1) agencé sur ledit bras de référence et un deuxième objectif à immersion (LO2) agencé sur ledit bras objet, lesdits bras de référence et objet étant disjoints

8. Appareil selon l'une des revendications 1 à 5 dans lequel ledit microscope interférométrique est choisi parmi un microscope de Michelson et un microscope de Mirau, comprenant un objectif unique (LO).

9. Appareil selon l'une des revendications 1 ou 2 dans lequel ledit microscope interférométrique est un microscope de Mirau comprenant un objectif unique (LO), ledit miroir de référence (MR) et ledit séparateur de faisceau (SF) étant solidaires dudit objectif et alignés le long de son axe optique, ledit système d'actionnement étant configuré pour provoquer un déplacement relatif, parallèle audit axe optique, dudit microscope interférométrique par rapport à l'objet à observer, un milieu d'immersion (IM) étant agencé, d'une part entre ledit séparateur de faisceau et l'objet à observer et d'autre part entre ledit séparateur de faisceau et le miroir de référence.

10. Appareil selon l'une des revendications précédentes dans lequel ladite source de lumière polychromatique (DSL, FMM) est spatialement cohérente et ledit système de filtrage confocal comprend un système optique astigmatique (LC, LE) agencée entre ladite source de lumière polychromatique et le séparateur de faisceau (SF), une lentille (LF) agencée devant ledit capteur optique monodimensionnel (CIM) et le capteur optique monodimensionnel lui-même, agencé dans un plan de mise au point de ladite lentille.

11. Procédé de tomographie optique comprenant les étapes suivantes :

a) procurer une source de lumière polychromatique (SLM) ;

b) utiliser un séparateur de faisceau (SF) pour diriger une première fraction de lumière émise par ladite source suivant un premier trajet, dit trajet de référence (TREF), et une seconde fraction de lumière émise par ladite source suivant un second trajet, dit trajet objet (TROB) ;

c) utiliser un objectif (LO2) coopérant avec un système de filtrage spatial confocal monodimensionnel (FS) pour focaliser ladite seconde fraction de lumière de façon à éclairer un objet (OBJ) semi-transparent à observer selon une ligne, dite ligne d'observation, située dans un plan de mise au point dudit objectif, et pour collecter la lumière rétrodiffusée par ledit objet ainsi éclairé ;

d) utiliser ledit objectif, ou un autre objectif (LO1), pour focaliser ladite première fraction de lumière sur un miroir de référence (MR) agencé sur ledit trajet de référence, et pour collecter la lumière réfléchie par ledit miroir ;

e) utiliser ledit séparateur de faisceau (SF) pour combiner la lumière rétrodiffusée par ledit objet avec la lumière réfléchie par ledit miroir et la diriger vers un capteur optique monodimensionnel (CIM) ;

f) utiliser ledit système de filtrage spatial confocal monodimensionnel (FS) pour former une image monodimensionnelle de ladite ligne d'observation sur ledit capteur ;

g) utiliser un système d'actionnement (PR, TR1, TR2, TR3, TRO) pour déplacer ladite ligne d'observation parallèlement à un axe optique dudit objectif (LO2) de façon à réaliser un balayage unidirectionnel d'un objet à observer sur ledit trajet objet, tout en maintenant une différence de chemin optique nulle entre ledit trajet de référence et ledit trajet objet ; et

h) utiliser un processeur (PR) pour reconstituer une image bidimensionnelle d'une section dudit objet à observer, orientée parallèlement audit axe optique, à partir d'une pluralité d'images interférométriques monodimensionnelles acquises par ledit capteur en correspondance de positions différentes de ladite ligne d'observation au cours dudit balayage unidirectionnel.

12. Procédé selon la revendication 11 dans lequel ladite étape f) comprend également une sélection de la lumière provenant de ladite ligne d'observation (LDO).

13. Procédé selon l'une des revendications 11 ou 12 dans lequel ladite étape g) est mise en œuvre en provoquant un déplacement relatif, parallèle audit axe optique, dudit objet à observer par rapport audit microscope interférométrique sans modifier les longueurs optiques dudit bras de référence et dudit bras objet.

14. Procédé selon l'une des revendications 11 ou 12 dans lequel ladite étape g) est mise en œuvre en déplaçant l'objectif dans un plan de mise au point duquel se trouve ladite ligne d'observation et en modifiant la longueur optique dudit bras de référence de manière à maintenir nulle la différence de chemin optique entre ledit premier trajet et ledit second trajet.

15. Procédé selon l'une des revendications 11 à 14 comprenant également une étape i) de compensation des modifications de la dispersion induites par le déplacement de la ligne d'observation à l'intérieur dudit objet à observer au cours dudit balayage unidirectionnel.

16. Procédé selon l'une des revendications 11 à 15, dans lequel ledit objet à observer est un tissu biologique.

**Patentansprüche**

1. Optische Tomographievorrichtung, umfassend:

  - eine polychromatische Lichtquelle (SLP);
  - einen eindimensionalen optischen Sensor (CIM);
  - ein interferometrisches Mikroskop (MI), umfassend: einen ersten Arm (BREF), bezeichnet als Referenzarm, an dessen Ende ein Referenzspiegel (MR) angeordnet ist; einen zweiten Arm (BOBJ), bezeichnet als Objektarm; einen Strahlteiler (SF), der den ersten und zweiten Arm mit der polychromatischen Lichtquelle und dem Sensor koppelt; und wenigstens ein Objektiv (LO1, L02, LO), wobei der Referenzspiegel in einer in dem Referenzarm angeordneten Fokusebene des Objektivs oder eines der Objektive (LO, LO1) angeordnet ist;
  - ein eindimensionales konfokales räumliches Filtersystem (FE, FS, CIM), das mit der polychromatischen Lichtquelle zusammenwirkt, um ein zu beobachtendes Objekt (OBJ), das in dem Objektarm (BOBJ) angeordnet ist, zu beleuchten, wobei eine Linie, bezeichnet als Beobachtungslinie (LDO), in einer in dem Objektarm angeordneten Fokusebene des Objektivs oder eines der Objektive (LO, L02) liegt, und um ein eindimensionales Bild der Beobachtungslinie auf dem Sensor zu erzeugen;

  **dadurch gekennzeichnet, dass** die Vorrichtung ferner umfasst:

  - ein Aktuatorsystem (PR, TR1, TR2, TR3), welches dazu konfiguriert ist, die Beobachtungslinie parallel zu einer optischen Achse, die sich entlang des Objektarms erstreckt, des Objektivs oder eines der Objektive (LO, L02) so zu verschieben, dass eine eindirektionale Abtastung des Objekts durchgeführt wird, wobei eine optische Wegdifferenz von Null zwischen einem ersten Weg, der von dem Strahlteiler zu dem Referenzspiegel und entlang des Referenzarms zurück verläuft, und einem zweiten Weg, der von dem Strahlteiler zu der Beobachtungslinie und entlang des Objektarms zurück verläuft, beibehalten wird; und
  - einen Prozessor (PR), der programmiert oder konfiguriert ist, ein zweidimensionales Bild eines Abschnitts des zu beobachtenden Objekts, der parallel zu der optischen Achse, die sich entlang des Objektarms erstreckt, des Objektivs (LO, L02) orientiert ist, aus mehreren eindimensionalen interferometrischen Bildern zu rekonstruieren, die entsprechend den verschiedenen Positionen der Beobachtungslinie während der eindirektionalen Abtastung von dem Sensor erfasst wurden.

2. Vorrichtung nach Anspruch 1, wobei das eindimensionale konfokale räumliche Filtersystem auch angeordnet ist, um das von dem Objekt zurückgestreute und von der Beobachtungslinie (LDO) stammende Licht auszuwählen.

3. Vorrichtung nach einem der voranstehenden Ansprüche, wobei das Aktuatorsystem dazu konfiguriert ist, eine Verschiebung des zu beobachtenden Objekts relativ zu dem interferometrischen Mikroskop parallel zu der optischen Achse des Objektivs (LO) oder eines in dem Objektarm angeordneten Objektivs (L02) hervorzurufen, ohne die optische Länge des Referenzarms und des Objektarms zu ändern.

4. Vorrichtung nach Anspruch 1 oder 2, wobei das Aktuatorsystem dazu konfiguriert ist, das Objektiv (L02), in dessen Fokusebene sich die Beobachtungslinie befindet, zu verschieben und die optische Länge des Referenzarms so zu ändern, dass die optische Wegdifferenz von Null zwischen dem ersten Weg und dem zweiten Weg erhalten bleibt.

5. Vorrichtung nach einem der voranstehenden Ansprüche, ferner umfassend: eine Dispersionskompensationsvorrichtung (DCD, IM), welche an mindestens einem von dem Objektarm und dem Referenzarm angeordnet ist, wobei das Aktuatorsystem ferner dazu konfiguriert ist, die Dispersionskompensationsvorrichtung während der eindirektionalen Abtastung zu bewegen.

**6.** Vorrichtung nach einem der voranstehenden Ansprüche, wobei das interferometrische Mikroskop ein Linnik-Mikroskop ist, welches ein in dem Referenzarm angeordnetes erstes Objektiv (LO1) und ein in dem Objektarm angeordnetes zweites Objektiv (L02) umfasst, wobei der Referenzarm und der Objektarm getrennt sind.

**7.** Vorrichtung nach Anspruch 3, wobei das interferometrische Mikroskop ein Linnik-Mikroskop ist, welches ein in dem Referenzarm angeordnetes erstes Immersionsobjektiv (LO1) und ein in dem Objektarm angeordnetes zweites Immersionsobjektiv (L02) umfasst, wobei der Referenzarm und der Objektarm getrennt sind.

**8.** Vorrichtung nach einem der Ansprüche 1 bis 5, wobei das interferometrische Mikroskop ein Michelson-Mikroskop oder ein Mirau-Mikroskop ist, welches ein einziges Objektiv (LO) umfasst.

**9.** Vorrichtung nach Anspruch 1 oder 2, wobei das interferometrische Mikroskop ein Mirau-Mikroskop ist, welches ein einziges Objektiv (LO) umfasst, wobei der Referenzspiegel (MR) und der Strahlteiler (SF) an dem Objektiv befestigt sind und an seiner optischen Achse ausgerichtet sind, wobei das Aktuatorsystem dazu konfiguriert ist, eine Verschiebung des interferometrischen Mikroskops relativ zu einem zu beobachtenden Objekt parallel zu der optischen Achse hervorzurufen, wobei ein Immersionsmedium (IM) einerseits zwischen dem Strahlteiler und dem zu beobachtenden Objekt und andererseits zwischen dem Strahlteiler und dem Referenzspiegel angeordnet ist.

**10.** Vorrichtung nach einem der voranstehenden Ansprüche, wobei die polychromatische Lichtquelle (DSL, FMM) räumlich kohärent ist und das konfokale Filtersystem ein zwischen der polychromatischen Lichtquelle und dem Strahlteiler (SF) angeordnetes astigmatisches optisches System (LC, LE), eine vor dem eindimensionalen optischen Sensor (CIM) angeordnete Linse (LF) und den eindimensionalen optischen Sensor selbst aufweist, welcher in einer Fokusebene der Linse angeordnet ist.

**11.** Optisches Tomographieverfahren, umfassend die folgenden Schritte:

a) Bereitstellen einer polychromatischen Lichtquelle (SLM);
b) Verwenden eines Strahlteilers (SF), um einen ersten Teil des von der Quelle emittierten Lichts entlang einer ersten Weges, bezeichnet als Referenzweg (TREF), und einen zweiten Teil des von der Quelle emittierten Lichts entlang eines zweiten Weges, bezeichnet als Objektweg (TROB), zu lenken;
c) Verwenden eines Objektivs (L02), das mit einem eindimensionalen konfokalen räumlichen Filtersystem (FS) zusammenwirkt, um den zweiten Teil des Lichts so zu fokussieren, dass ein zu beobachtendes halbtransparentes Objekt (OBJ) beleuchtet wird, wobei eine Linie, bezeichnet als Beobachtungslinie, in einer Fokusebene des Objektivs angeordnet ist, und um das von dem beleuchteten Objekt zurückgestreute Licht zu sammeln;
d) Verwenden des Objektivs oder eines anderen Objektivs (LO1), um den ersten Teil des Lichts auf einen in dem Referenzweg angeordneten Referenzspiegel (MR) zu fokussieren und um das von dem Referenzspiegel reflektierte Licht zu sammeln;
e) Verwenden des Strahlteilers (SF), um das von dem Objekt zurückgestreute Licht mit dem von dem Spiegel reflektierten Licht zu kombinieren und es zu einem eindimensionalen optischen Sensor (CIM) zu lenken;
f) Verwenden des eindimensionalen konfokalen räumlichen Filtersystems (FS), um ein eindimensionales Bild der Beobachtungslinie auf dem Sensor zu bilden;
g) Verwenden eines Aktuatorsystems (PR, TR1, TR2, TR3, TRO), um die Beobachtungslinie parallel zu einer optischen Achse des Objektivs (L02) zu verschieben, um eine eindirektionale Abtastung eines zu beobachtenden Objekts auf dem Objektweg durchzuführen, während eine optische Wegdifferenz von Null zwischen dem Referenzweg und dem Objektweg erhalten bleibt; und
h) Verwenden eines Prozessors (PR) zum Rekonstruieren eines zweidimensionalen Bildes eines Abschnitts des zu beobachtenden Objekts, der parallel zu der optischen Achse orientiert ist, aus mehreren eindimensionalen interferometrischen Bildern, die entsprechend verschiedenen Positionen der Beobachtungslinie während der eindirektionalen Abtastung von dem Sensor erfasst wurden.

**12.** Verfahren nach Anspruch 11, wobei der Schritt f) ferner das Auswählen des von der Beobachtungslinie (LDO) ausgehenden Lichts umfasst.

**13.** Verfahren nach Anspruch 11 oder 12, wobei der Schritt g) ausgeführt wird, indem eine Verschiebung des zu beobachtenden Objekts relativ zu dem interferometrischen Mikroskop parallel zur optischen Achse hervorgerufen wird, ohne die optische Länge des Referenzarms und des Objektarms zu ändern.

**14.** Verfahren nach Anspruch 11 oder 12, wobei der Schritt g) ausgeführt wird, indem das Objektiv in einer Fokussierebene verschoben wird, auf der sich die Beobachtungslinie befindet, und indem die optische Länge des Referenzarms so geändert wird, dass die optische Wegdifferenz von Null zwischen dem ersten und dem zweiten Weg erhalten bleibt.

15. Verfahren nach einem der Ansprüche 11 bis 14, ferner umfassend einen Schritt i) der Kompensation der durch die Verschiebung der Beobachtungslinie ins Innere des zu beobachtenden Objekts hervorgerufenen Änderungen der Dispersion während der eindirektionalen Abtastung.

16. Verfahren nach einem der Ansprüche 11 bis 15, wobei das zu beobachtende Objekt ein biologisches Gewebe ist.

**Claims**

1. An optical tomography apparatus comprising:

   - a polychromatic light source (SLP);
   - a one-dimensional optical sensor (CIM);
   - an interferometric microscope (MI) comprising: a first arm (BREF), called the reference arm, at the end of which a so-called reference mirror (MR) is arranged; a second arm (BOBJ), called the object arm; a beam splitter (SF) coupling said first and second arms to said polychromatic light source and to said sensor; and at least one lens (LO1, LO2, LO), said reference mirror being arranged in a focusing plane, located in the reference arm, of said or of one said lens (LO, LO1);
   - a one-dimensional confocal spatial filtering system (FE, FS, CIM), cooperating with said polychromatic light source to illuminate an object to be observed (OBJ), arranged in said object arm (BOBJ), along a line, called the observation line (LDO), lying in a focusing plane, located in the object arm, of said or of one of said lens (LO, L02), and to form a one-dimensional image of said observation line on said sensor; **characterized in that** it also comprises:

      - an actuating system (PR, TR1, TR2, TR3) configured to move said observation line parallel to an optical axis extending along the object arm of said or of one of said lens (LO, L02) so as to perform a one-way scan of said object, while maintaining a zero optical path difference between, on the one hand, a first path from said beam splitter to said reference mirror and back through said reference arm and, on the other hand, a second path from said beam splitter to said observation line and back through said object arm; and

      - a processor (PR) programmed or configured to reconstruct a two-dimensional image of a section of said object to be observed, oriented parallel to said optical axis extending along the object arm of said lens (LO, L02), from a plurality of one-dimensional interferometric images acquired by said sensor in correspondence with different positions of said observation line during said one-way scan.

2. The apparatus according to claim 1, wherein said one-dimensional confocal spatial filtering system is also arranged to select the light backscattered by said object and coming from said observation line (LDO).

3. The apparatus according to one of the preceding claims, wherein said actuating system is configured to cause relative movement, parallel to said optical axis of said lens (LO) or of one said lens placed in the object arm (L02), of said object to be observed with respect to said interferometric microscope, without changing the optical lengths of said reference arm and said object arm.

4. The apparatus according to one of claims 1 or 2, wherein said actuating system is configured to move the lens (L02) in the focusing plane of which said observation line is located and to change the optical length of said reference arm so as to maintain the optical path difference between said first path and said second path at zero.

5. The apparatus according to one of the preceding claims, further comprising a dispersion compensation device (DCD, IM) arranged on at least one of said object arm and said reference arm, said actuating system being configured to also act on said dispersion compensation device during said one-way scan.

6. The apparatus according to one of the preceding claims, wherein said interferometric microscope is a Linnik microscope, comprising a first lens (LO1) arranged on said reference arm and a second lens (L02) arranged on said object arm, said reference and object arms being disjoint.

7. The apparatus according to claim 3, wherein said interferometric microscope is a Linnik microscope, comprising a first immersion lens (LO1) arranged on said reference arm and a second immersion lens (L02) arranged on said object arm, said reference and object arms being disjoint.

8. The apparatus according to one of claims 1 to 5, wherein said interferometric microscope is selected from a Michelson microscope and a Mirau microscope, comprising a single lens (LO).

9. The apparatus according to one of claims 1 or 2, wherein said interferometric microscope is a Mirau microscope comprising a single lens (LO), said reference mirror (MR) and said beam splitter (SF) being integral with said lens and aligned along its optical axis, said actuating system being configured to cause a relative displacement, parallel to said optical axis, of said interferometric microscope with respect to the object to be observed, an immersion medium (IM) being arranged, on the one hand, between said beam splitter and the object to be observed and, on the other hand, between said beam splitter and the reference mirror.

10. The apparatus according to one of the preceding claims, wherein said polychromatic light source (DSL, FMM) is spatially coherent and said confocal filtering system comprises an astigmatic optical system (LC, LE) arranged between said polychromatic light source and the beam splitter (SF), a lens (LF) arranged in front of said one-dimensional optical sensor (CIM) and the one-dimensional optical sensor itself, arranged in a focusing plane of said lens.

11. An optical tomography method, comprising the following steps:

    a) providing a polychromatic light source (SLM);
    b) using a beam splitter (SF) to direct a first fraction of light emitted by said source along a first path, called the reference path (TREF), and a second fraction of light emitted by said source along a second path, called the object path (TROB);
    c) using a lens (LO2) cooperating with a one-dimensional confocal spatial filtering system (FS) to focus said second fraction of light so as to illuminate a semi-transparent object (OBJ) to be observed along a line, called the observation line, located in a focusing plane of said lens, and to collect the light backscattered by said object thus illuminated;
    d) using said lens, or another lens (LO1), to focus said first fraction of light onto a reference mirror (MR) arranged on said reference path, and to collect the light reflected by said mirror;
    e) using said beam splitter (SF) to combine light backscattered from said object with light reflected from said mirror and direct it to a one-dimensional optical sensor (CIM);
    f) using said one-dimensional confocal spatial filtering system (FS) to form a one-dimensional image of said observation line on said sensor;
    g) using an actuating system (PR, TR1, TR2, TR3, TRO) for moving said observation line parallel to an optical axis of said lens (L02) so as to perform a one-way scan of an object to be observed on said object path, while maintaining a zero optical path difference between said reference path and said object path; and
    h) using a processor (PR) to reconstruct a two-dimensional image of a section of said object to be observed, oriented parallel to said optical axis, from a plurality of one-dimensional interferometric images acquired by said sensor in correspondence with different positions of said observation line during said one-way scan.

12. The method according to claim 11, wherein said step f) also comprises selecting the light from said observation line (LDO).

13. The method according to one of claims 11 or 12, wherein said step g) is carried out by causing a relative displacement, parallel to said optical axis, of said object to be observed with respect to said interferometric microscope without changing the optical lengths of said reference arm and said object arm.

14. The method according to one of claims 11 or 12, wherein said step g) is carried out by moving the lens in a focusing plane in which said observation line is located and changing the optical length of said reference arm so as to maintain the zero optical path difference between said first path and said second path.

15. The method according to one of claims 11 to 14, further comprising a step i) of compensating for changes in dispersion induced by the displacement of the observation line within said object to be observed during said one-way scan.

16. The method according to one of claims 11 to 15, wherein said object to be observed is a biological tissue.

FIG.1A

FIG.1B

FIG.2A

FIG.2B

FIG.3A

FIG.3B

FIG.4A

FIG.4B

LO

MR

SF

IM

OBJ

**FIG.5A**

LO

IM

MR

OBJ

**FIG.5B**

LO

MR

SF

IM

OBJ

HO

**FIG.5C**

LO

MR

SF

IM

OBJ

**FIG.5D**

LO

IM

MR

SF

OBJ

HO

**FIG.5E**

LO

MR

SF

IM

OBJ

HO

**FIG.5F**

FIG.5G

FIG.6

EP 3 084 345 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1364181 A
- EP 2447754 A
- EP 1586931 A

**Littérature non-brevet citée dans la description**

- **A. F. FERCHER.** Optical coherence tomography - principles and applications. *Reports on Progress in Physics,* 2003, vol. 66, 239-303
- **A. DUBOIS ; K. GRIEVE ; G. MONERON ; R. LECAQUE ; L. VABRE ; A.C. BOCCARA.** Ultra-high-resolution full-field optical coherence tomography. *Applied Optics,* 2004, vol. 43, 2874
- **S. KIM et al.** Simultaneous measurement of refractive index and thickness by combining low-coherence interferometry and confocal optics. *Optics Express,* 14 Avril 2008, vol. 16 (8), 5516
- **YU CHEN et al.** High-resolution line-scanning optical coherence microscopy. *Optics Letters,* 15 Juillet 2007, vol. 32 (14), 1971-1973